# EUROPEAN PATENT APPLICATION

(11) **EP 2 090 320 A1**
(43) Date of publication of application: **19.08.2009**
(21) Application number: 08002867.3
(22) Date of filing: 15.02.2008
(51) Int. Cl.: A61K 39/395, C07K 16/28

(54) **Ligands of the Natural Killer (NK) cell surface marker CD27 and therapeutic uses thereof**

(71) Applicant: Helmholtz-Zentrum für Infektionsforschung GmbH, 38124 Braunschweig (DE)
(72) Inventor: Weiss, Siegfried, 38118 Braunschweig (DE); Viegas, Nuno, 38102 Braunschweig (DE)
(74) Representative: Krauss, Jan

(57) **Abstract**

The present invention relates to the modulation of Natural Killer (NK) cells in vitro or in vivo through the use of activating or inhibiting ligands of CD27, such as antibodies, for a regulation of the immune response against several different diseases, such as viral infections, cancer, bacterial infections, sepsis as well as immunological diseases. In a preferred embodiment, the inventors were able to improve the host resistance against Influenza virus and cancer through the application of anti-CD27 antibodies.In a different setting the inventors were able to prevent the onset of a lethal bacterial sepsis by preventing the activation of NK cells via CD27. The invention furthermore relates to screening assays for ligands of the surface marker CD27.

## Description

The present invention relates to the modulation of Natural Killer (NK) cells in vitro or in vivo through the use of activating or inhibiting ligands of CD27, such as antibodies, for a regulation of the immune response against several different diseases, such as viral infections, cancer, bacterial infections, sepsis as well as immunological diseases. In a preferred embodiment, the inventors were able to improve the host resistance against Influenza virus and cancer through the application of anti-CD27 antibodies. In a different setting the inventors were able to prevent the onset of a lethal bacterial sepsis by preventing the activation of NK cells via CD27. The invention furthermore relates to screening assays for ligands of the surface marker CD27.

### Description

Natural Killer (NK) cells constitute a key frontline defence against a range of tumours and pathogens including intracellular bacteria, parasites and, most importantly, viruses. The antimicrobial mechanisms by which NK cells operate include both a direct cytotoxic response as well as production of cytokines and chemokines that modulate other cells of the immune system.

The conventional treatment of cancer is usually performed using radio- or chemotherapy. Viral infections are treated with several anti-viral drugs. Bacterial infections are usually treated with antibiotics. Nevertheless, several bacterial infections cannot be treated with antibiotics (as, for example, sepsis).

Septicaemia has become a leading cause of death for patients admitted to Intensive Care Unit in the USA and Europe (Linde-Zwirble and Angus 2004; Minino, Heron and Smith 2006). The rate of mortality is high and varies from 30% to 70% (Riedemann, Guo and Ward 2003). The common cause of death in sepsis is multiple organ dysfunction syndrome (MODS) and is usually the terminal result of the systemic inflammatory response syndrome (SIRS).

Bacterial sepsis was approached with antibodies or soluble molecules against cytokines like TNF-α and IL-6, but only with controversial results, and a beneficial use could not be proven. Lately, IFNy has also been proposed as a therapy against several pathologies. Recently, the use of IFN-α and IFN-γ has also become common against multiple sclerosis.

Blocking antibodies, inhibitors or antagonists against the main mediators of MODS such as IL-1, nitric oxide (NO), platelet activating factor, arachidonic acid metabolites, oxygen radicals, bradykinin, phosphodiesterase and C1 esterase gave conflicting results in randomized clinical trials, or even showed no overall benefit for the survival of septicaemic patients. Neutralizing the pleiotropic cytokine TNF-α had positive effects in some cases of sepsis due to Gram-negative infection but was detrimental to septicaemic patients suffering from Gram-positive bacterial sepsis. (van der Poll 2001; Vincent, Sun and Dubois 2002).

Most of the knowledge presently available on sepsis and infections is based on the research on animal models infected with Gram-negative bacteria, or treated with their components, like LPS and Lipid A. Models using undefined mixed bacterial populations like in caecal puncture sepsis are also commonly used (Buras, Holzmann, and Sitkovsky 2005; Lorente and Marshall 2005; Van Amersfoort, Van Berkel and Kuiper 2003). Until today, the role of Gram-positive bacteria in septic infections has been neglected. However, the study of Gram-positive bacteria in sepsis has become very urgent. Infections by such bacteria have increased in the last decades from 4-24 % to more than 50%. The main reason for this is the increased use of catheters and other invasive medical equipment and the use of chemotherapy or immunosuppressive drugs, and the emerge of AIDS. (Geerdes et al. 1992; Van Amersfoort, Van Berkel and Kuiper 2003).

Listeria (L.) monocytogenes is one of the most intensively studied Gram-positive bacterial pathogen. These bacteria are frequently used as a model to study immune effector mechanisms as well as evasion strategies of intracellular bacteria and sepsis. L. monocytogenes, after intravenous (i.v.) administration, is removed within minutes from circulation through phagocytic cells of spleen and liver. Innate mechanisms of the immune system then keep the proliferation of the bacteria under control until specific T cells have expanded sufficiently to sterily clear the infection. Any incapacitation of the innate defense mechanisms that will compromise the initial control of the bacteria will lead to fatal sepsis after a short period of time. During this period, neutrophilic granulocytes and macrophages are believed to be the major effector cells. They are recruited and activated by cytokines produced by myeloid and lymphoid cells as well as by NK cells (Pamer 2004).

Early resistance to L. monocytogenes infection was until now attributed to the production of interferon-γ (IFN-γ) by natural killer cells that promotes the activation of macrophages and dendritic cells (Pamer 2004). Contradictory to this data is that the depletion of NK1.1⁺ cells resulted in reduction of bacterial burden in the spleen of infected mice (Teixeira and Kaufmann 1994). In order to resolve this contradiction, the inventors had decided to study the early immune events of a Listeria infection in murine listeriosis, paying special attention to the NK cells.

The current treatments of cancer have low rates of success and strong secondary effects reduce the quality of life of the patients.

The treatment of viral infection is doubtful, and can lead to the appearance of strains insensitive to the drugs used. Antiviral drugs are also always associated with strong secondary effects. For chronic viral infections, an efficient treatment is not available.

The current therapy for bacterial infections is antibiotics. The appearance of several antibiotic resistant bacteria makes scientists to think in other type of therapies. In addition, in cases where bacterial infection results in sepsis, the use of antibiotics is not recommended. In these cases, it is thought that the modulation of the immune system provides the best treatment.

The modulation of the immune system using monoclonal antibodies against cell surface molecules from cell from the immune system has been already described for some cases. As one example, the use of CD70 antibodies for a treatment of immune disorders, cancer and AIDS has been proposed in WO 2006/113909, but not for other bacterial or viral diseases, and NKG2D antibodies were described for the treatment of cancer and viral infection, but not bacterial infections and immune disorders.

The viral strain H5N1 is the most recent example of a influenza strain that that is capable of infecting several species of animals along a wide area (panzootic virus) and that can recombinate with other influenza strains, originating a highly virulent virus. This strain is rapidly spreading globally after its first outbreak in Asia, having been recently detected in Europe.

The fast development, production and distribution of a human vaccine against the pandemic influenza strain is extremely expensive, demanding and involves several months time lapse until the vaccine is available to the general public. The only current alternative treatments against new highly infectious influenza strains are the viral neuraminidase inhibitors Oseltamivir and Zanamivir that are respectively commercialized with the names Tamiflu and Relenza. Nevertheless, this drugs have been associated with strong secondary effects on the patients to which they where administrated and their continuous use can also lead to the appearance of neuraminidase inhibitors resistant virus strains. (Tamiflu product information; FDA Reports on Relenza) The conventional neuraminidase inhibitor treatments present a large range of undesirably secondary effects and limitations on the number of available doses.

US 5,573,924 and EP 0 662 077 generally provide the CD27 ligand (CD27L) that binds to the CD27 receptor, isolated DNA encoding the CD27L protein, expression vectors comprising the isolated DNA, and a method for producing CD27L by cultivating host cells containing the expression vectors under conditions appropriate for expression of the CD27L protein. Antibodies directed against the CD27L protein or an immunogenic fragment thereof are also disclosed. There is no disclosure regarding the link of CD27L with diseases.

To the knowledge of the inventors, there is no furthermore no disclosure regarding the use of CD27 antibodies, and treatment of sepsis by modifying the activity ofNK cells.

In view of the above, it is an object of the present invention, to provide novel cell surface markers ofNK cells that regulate the activity of NK cells in order to fully exploit the therapeutic potential of these cells. Furthermore, said novel cell surface markers of NK cells can be used as tools in order to screen for new medicaments for the prevention and/or treatment of diseases that can be influenced by modifying the activities ofNK cells.

According to a first aspect thereof, the object of the present invention is solved by providing the use of a ligand of CD27 for the preparation of a medicament for the prevention or treatment of cancer, viral and bacterial infections as well as immune disorders through the activation, depletion or inhibition of NK cells. Preferred is a use according to the present invention, wherein said ligand is activating NK cells, and is for the prevention or treatment of viral infections, for example influenza virus.

In the context of the present invention, it was found that the activation of NK cells influences dramatically the priming of an efficient immune response against, for example, influenza virus, promoting the efficient clearance of the virus from the host system.

Preferred is further a use according to the present invention, wherein said activation of said NK cells furthermore promotes the efficient clearance of the virus from the host system.

Preferred is further a use according to the present invention, wherein said ligand is depleting or inhibiting NK cells, and is for the prevention or treatment of diseases selected from the group of an infection caused by Gram-positive bacteria, such as Listeria monocytogenes, the treatment or onset of diabetes, graft versus host disease, and immune disorders and sepsis. Furthermore, it was found that the activation of NK cells is detrimental in case of the priming of an immune response against an infection caused by the Gram-positive bacteria Listeria monocytogenes.

Without wanting to be bound by theory, it seems that the balance of activation/inhibition of NK cells during an immune response can influence dramatically the outcome of infectious diseases and other pathologies where NK cells are involved, like sepsis, cancer, diabetes, graft versus host (GvH) and other immune disorders.

According to the experiments of the inventors, the outcome of disease greatly depends on the fine tuning of the immune response. NK cells are known for their immune-modulatory properties. The inventors could prove (see table 1) that using blocking antibodies or soluble molecules against NK cell surface molecules or their ligands the specific blockage of activating signals of NK cells is beneficial on a case of infection and sepsis caused by Gram-positive bacteria. In accordance with this data, a specific blockage of activating receptors on the surface of NK cells is beneficial in cases where the over-activation of the immune system may result in a pathology; as for example the onset of diabetes, graft versus host disease and immune disorders and sepsis.

The inhibition of NK cell over-stimulation can also be achieved by using monoclonal antibodies or soluble ligands against inhibitory receptors thus preventing activation signals on the cell surface of these cells (see table 2).

In contrast the inventors could also produce data that the clearance of cancer, viral particles and other microorganisms from infected hosts can be improved dramatically by providing specific costimulation to NK cells using activating antibodies or soluble molecules against cell surface activating receptors, like CD27. These findings led to a new therapy approach against influenza since it is independent of the variant of the virus.

The specific activation of NK cells can also be used as therapy against other infectious agents as well as diverse forms from cancer.

In cases were activation or inhibition of NK cells is not possible, the depletion of these cells using specific antibodies will have a positive effect on the treatment of pathologies.

**Table 1. Table with a list from several activating receptors expressed on the cell surface of NK cells and respective molecular ligands**

| Activating receptor | Motif/Adaptor | Class | Ligand |
|---|---|---|---|
| CD16 | ITAM/FcyR | IgSF | Immune complexes |
| CD25 | ? | CytoR | IL-2 |
| CD27 | TRAF | TNFRSF | CD70 |
| CD28 | YXXM/PI3K | IgSF | CD80, CD86 |
| CD69 | ? | C-lectin | ? |
| CD94/NKG2C, E | ITAM/DAP12 | C-lectin | HLA-E, Qa-1b |
| CD122 | JAK1, 3/STAT5a, 5b | CytoR | IL-2, IL-5 |
| CD161 | ? | | Clr-g (NKR-P1F) |
| CD226 | ? | | CD112, CD 155 |
| CD244 | TXYXXV-I/SAP, Fyn | SLAM | CD48 |
| NKG2D | YINM/DAP10, PI3K | C-lectin | MICA, B, ULBs, Rae1s |
| KIR2S, KIR3S | ITAM/DAP12 | IgSF | HLA class I |
| Type I IFN Receptor | JAK1, Tyk2/STAT1, 4 | CytoR | Type I Interferons |
| NCR (NKp30, 44, 46) | ITAM/FcyR, CD3ζ, DAP 12 | IgSF | Viral hemagglutinins |
| ILT-1 | ITAM/FcyR, DAP12 | IgSF | ? |

**Table 2. Table with a list from several inhibitory receptors expressed on the cell surface of NK cells and respective molecular ligands**

| Inhibitory receptor | Motif/Adaptor | Class | Ligand |
|---|---|---|---|
| CD85 | IgSF | ITIM/SHP-1 | HLA-A,-B,-G |
| CD94/NKG2A | C-lectin | ITIM/SHP-1,-2 | HLA-E |
| CD244 | SLAM | TXYXXV-I/SAP, Fyn | CD48 |
| KIR2DL, KIR3DL | IgSF | ITIM/SHP-1, -2 | HLA class I |
| TGF-βR | CytoR | Smad2 | TGF-β Family |
| IL-10R | CytoR | JAK2, Tyk2/STAT3 | IL-10 |

The inventors also know from their experiments that the neutralization of type I interferons (IFN-γ, IFN-α) by antibodies or soluble molecules is able to reduce the activation status of NK cells and can also used as a therapy. The high production of type II interferon (IFN-γ) by NK cells will later on lead to a state of immune suppression that will result on a uncontrollable disease proliferation.

In summary, the modulation of the activation or inhibition of NK cells activity via cell surface receptors during the onset of bacterial or viral infection, cancer, diabetes or immune disorders (using monoclonal antibodies, small molecules or specific ligands), strongly influence the global immune response and can be used has a new immunotherapy.

The immune modulation from NK cells through their receptors or the use of soluble molecules (as type I interferons) leads to a regulation and fine tuning from the immune response against several diseases with high efficiency and low side effects.

Since the immune system adapts in response to a stimulus the therapy against several pathologies will be optimal. Another advantage is that there will be no danger of the appearance microorganisms with resistance to this treatment.

During infections of mice by Listeria monocytogenes, NK 1.1⁺ cells could be shown to be responsible for exacerbation of the disease. Mice depleted of NK 1.1⁺ cells by anti-NK1.1 antibodies harboured lower numbers of bacteria in spleen and liver and survived lethal infections. A contribution of NKT cells and the recently described IKDC to the adverse effects could be excluded. Rather, CD27^{hi} NK cells appeared to be responsible by producing high amounts of IFN-γ. Inhibiting IFN-γ or blocking signalling via CD27 resulted in an increase of neutrophils and macrophages in the infected spleen, which most likely caused the control of listerial expansion. The massive tissue destruction in spleen that is usually accompanied with L. monocytogenes infection was avoided under these circumstances. Thus, IFN-γ producing NK cells that are often advantageous in defense reactions against pathogens are detrimental in listeriosis. It is assumed that such effects can be expanded to other models of sepsis by Gram-positive bacteria, and thus inhibiting of signalling via CD27 or blocking of IFN-γ appears to be a general therapy for intervention of this fatal conditions.

The inventors' data suggest that during L. monocytogenes infection a subpopulation of NK cells produces excessive amounts of IFNy after an over-stimulation via CD27/CD70. The high level of this pleiotropic cytokine results in a deficient recruitment of granulocytes, macrophages and B cells into the spleen leading to an inefficient bacterial control and exacerbation of the infection.

Further preferred is a use according to the present application, wherein said ligand of CD27 is selected from a CD27-specific antibody, an CD27-binding fragment thereof, a CD27-binding peptide, and a CD27-interacting substance. Preferably, said antibody is a human, humanized, mouse or chimeric antibody.

Further preferred is a use according to the present application, wherein said ligand is administered systemically and/or administered locally. Further preferred is a use, wherein said ligand is for administration in combination with other chemotherapeutically active substances, such as antibiotics or anti-cancer chemotherapeutics (as also described below).

According to yet another aspect thereof, the object of the present invention is solved by providing a method for screening for CD27 ligands comprising the steps of: a) incubating a cell expressing CD27 with a putative ligand, b) measuring, if a binding between CD27 and said putative ligand occurs, c) in the case of a binding of said ligand to CD27 is measured, measuring, if said binding between CD27 and said identified ligand also leads to a CD27-mediated activation, depletion or inhibition ofNK cells.

This method is suitable for the determination of compounds that can interact with CD27 on, preferably, NK cells, and to identify, for example, inhibitors, activators, competitors or modulators of CD27, in particular inhibitors, activators, competitors or modulators of CD27. Preferred is a method according to the present invention, wherein said screening takes place in vitro or in vivo. Further preferred is a method according to the present invention, wherein said ligand of CD27 is selected from a peptide library, a combinatory library, a cell extract, in particular a plant cell extract, a "small molecular drug", a bacterial metabolite, a phage display, an antibody or fragment thereof, a protein and/or a protein fragment.

The term "contacting" in the present invention means any interaction between the potentially binding substance(s) with CD27, whereby any of the two components can be independently of each other in a liquid phase, for example in solution, or in suspension or can be bound to a solid phase, for example, in the form of an essentially planar surface or in the form of particles, pearls or the like. In a preferred embodiment, a multitude of different potentially binding substances are immobilized on a solid surface like, for example, on a compound library chip and CD27 (or a functional part thereof or a NK cell expressing CD27) is subsequently contacted with such a chip.

The CD27 employed in a method of the present invention can be a full length protein or a fragment with N/C-terminal and/or internal deletions. Preferably the fragment is either an N-terminal fragment or a C-terminal fragment comprising the cytoplasmic region, depending on whether potentially interacting compounds are sought that specifically interact with the N- or C-terminal fragment.

The potentially binding substance, whose binding to CD27 is to be measured, can be any chemical substance or any mixture thereof. For example, it can be a substance of a peptide library, a combinatory library, a bacterial metabolite, a phage display, a cell extract, in particular a plant cell extract, a "small molecular drug", a protein and/or a protein fragment.

Measuring of binding of the compound to CD27 can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either CD27 or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to CD27 or CD27 fragments. The effect of the binding of the compound or the activity of CD27 can also be measured indirectly, for example, by assaying the activity of the CD27-NK cell after binding, and the like.

As a further step after measuring the binding of a potentially interacting compound and after having measured at least two different potentially interacting compounds at least one compound can be selected, for example, on grounds of the measured binding activity or on grounds of the detected increase or decrease ofNK cell activity and/or CD27 expression.

The thus selected binding compound is then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with CD27 and subsequently binding of the modified compounds to the CD27 polypeptide is measured. In this step, both the binding per se can be measured and/or the effect of the function of the CD27 like, e.g. the activity of the NK cells expressing the polypeptide can be measured. If needed, the steps of selecting the binding compound, modifying the binding compound, contacting the binding compound with a CD27 polypeptide and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity of the CD27 polypeptide(s), and/or the NK cells expressing said polypeptide.

In a further embodiment of the method of the present invention, the interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills.

Another aspect of the present invention relates to a method for the production of a pharmaceutical formulation, comprising the steps of: a) performing a method according to the present invention, and b) formulating the identified ligand for CD27 with a pharmaceutically acceptable carrier and/or excipient.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used.

In another aspect of the present invention, the screening tool for a ligand for CD27 for treating or preventing cancer, viral and bacterial infections as well as immune disorders through the activation, depletion or inhibition of NK cells, is an NK cell which is recombinantly expressing CD27. The expression constructs can be present extrachromosomally or integrated into the chromosome. The CD27 polypeptide can be expressed in the form of a fusion protein, for example together with an enzymatically active moiety as reporter-construct, in order to be able to detect the expression product.

In another aspect of the present invention, the screening tool for a ligand for CD27 for treating or preventing cancer, viral and bacterial infections as well as immune disorders through the activation, depletion or inhibition of NK cells, is a non-human transgenic mammal whose NK cells express CD27. Preferred is a transgenic mouse, rat, pig, goat or sheep. Methods to produce these non-human transgenic mammals are well known to the person of skill in the art

Yet another aspect of the present invention is directed at a pharmaceutical composition for treating or preventing cancer, viral and bacterial infections as well as immune disorders through the activation, depletion or inhibition of NK cells, obtainable by a method according to the method as above. Another aspect of the present invention relates to a method or use as above, wherein the pharmaceutical composition further comprises additional pharmaceutically active ingredients that modulate the diseases to be treated.

Another aspect of the present invention relates to a method for treating or preventing cancer, viral and bacterial infections as well as immune disorders through the activation, depletion or inhibition of NK cells, comprising administering to a subject in need thereof an effective amount of a pharmaceutical composition according to the present invention. Preferably, an active agent is administered in form of a pharmaceutical composition, such as an antibody, or a binding compound. Preferably, said patient is a human being or a domesticated animal. Treating is meant to include, e.g., preventing, treating, reducing the symptoms of, or curing the disease or condition.

An "effective amount" is an amount of the compound(s) as mentioned above that a) acts on the activity of NK cells via CD27 as analysed, and which alleviates symptoms as found for the disease. Alleviating is meant to include, e.g., preventing, treating, reducing the symptoms of, or curing the disease or condition.

The cytokine IFN-γ was always considered to be fundamental for the clearance of bacterial infections and has been accepted as an indicator for good prognosis for septicaemic patients. Similarly, it has been a dogma for a long time that in murine listeriosis IFN-γ produced by NK cells is required for the initial control of the bacteria (M. Mielke 1992). Therefore, it was unexpected that depletion ofNK 1.1 cells ameliorates the infection of mice by L. monocytogenes. In the present invention, the inventors could define the population of NK 1.1⁺ cells that is responsible for the adverse effect, and thus suggests a way by which such cells are activated. In addition, the inventors could unravel how the detrimental effect is exerted. First, by excluding other NK 1.1⁺ cell populations, the inventors could define NK1.1^{hi}NKG2D⁺CD27^{hi} cells as the population that is responsible for the exacerbation of Listeria infection.

The CD27 molecule itself acted as the receptor for stimulation of NK cells. This is in line with the findings of Takeda et al. (see above). Using monoclonal antibodies Takeda et al. could demonstrate that stimulation via CD27 induces the proliferation of NK cells and triggers them to produce IFN-γ. Interestingly, proliferation and IFN-γ production was enhanced when CD27 and NK 1.1 were stimulated simultaneously. The ligand of CD27 - CD70 - is upregulated during Listeria infection on T, B and dendritic cells.

A single injection of anti-CD70 antibody was sufficient to block the synergy and to reduce the stimulation level of the NK cells. Obviously, this allowed the survival of infected mice. We would expect to find similar results by blocking signalling via the NKG2D receptor. The absence such synergistic stimulation could also explain why residual CD27^{hi} NK1.1⁺ cells in anti-NK1.1 depleted mice exhibited hardly any IFN-γ producing cells. Surface expression of NK1.1 and NKG2D was lower in these mice, hence, preventing synergistic stimulation.

Recently, it could be shown that the absence of Type I IFN also ameliorated the course of infection by L. monocytogenes. This was claimed to be due to the induction of apoptosis of T cells and possibly also of macrophages in the early stage of listeriosis. On the other hand, it was recently shown that during Listeria infection type - I IFN is involved in the induction of IFN-γ by CD4 cells. NK cells were not be considered in this study but one is tempted to speculated that Type I IFN might also synergize with the co-stimulatory receptors mentioned above to overstimulate IFN-γ.

Overstimulation of NK1.1^{hi}NKG2D⁺CD27^{hi} cells resulted in amounts of IFN-γ that were obviously detrimental for the animals. Blocking of this cytokine led to survival of the animals. The effect of overproduction of this cytokine was a significant reduction of neutrophils, macrophages and B cells in the spleen of infected mice. The granulocyte population was affected most. It decreased progressively after 24 hours in the spleen of control mice but remained constant in the spleen of mice treated with anti-NK 1.1 IFN-γ produced by NK cells can induce apoptosis of infected cells but is also known to interfere with the migration of myeloid cells. In addition, IFN-γ was shown to inhibit proliferation of B cell precursors (Arens et al. 2001). The inventors did not find a significant increase in numbers of apoptotic cells in the spleen after Listeria infection. Therefore, the inventors interpret their findings that the overproduction of IFN-γ leads to a reduced influx of myeloid and lymphoid cells into the spleen of normal mice resulting an impairment of bacterial clearance.

This opens the question why mice in which the IFN-γ gene has been inactivated are highly susceptible to listeriosis. This cytokine might be essential for activation of effector cells in the later stages of listeriosis. Depletion by antibodies will only transiently deplete IFN-γ. Thus, it will be present at the time point where it is beneficial. Alternatively, antibodies will be able to deplete systemic IFN-γ but not local IFN-γ.

The accumulation of large numbers of dead cells, especially CD11b⁺, observed in the spleen of normal mice due to overproduction of IFN-γ gave rise to large necrotic areas. Most likely the reduced number of granulocytes and macrophages found during listeriosis is not sufficient to clear the elevated number of cells that had died during host reaction. The high number of dead cells might also be responsible for induction of anti-inflammatory cytokines. This might further inhibit anti-listerial defense reactions and add to the final onset of death by MODS.

In the light of the inventors' data one could speculate why splenectomized mice or mice deficient in T and B cells RAG2^{-/-}) are more resistant than normal mice to Listeria infection during the early phase of the innate immune response, showing a lower levels of apoptosis and of production of the anti-inflammatory cytokine interleukin 10 (IL-10) (Carrero, Calderon and Unanue 2006; Kuranaga et al. 2005). The inventors now know that Rag2^{-/-}, have no B and T cells on the primary site of Listeria inflammation, the spleen. The absence of T and B cells results on a reduction of the number of CD70 molecules available after infection on the spleen. By this way the stimulation ofNK cells via CD27 is eliminated, and there is no risk from a CD27^{high} NK cell overstimulation. The same is true for splenectomized mice were no more T and B cell areas are present near the main local of infection that is the spleen.

Detrimental effects elicited by IFN-γ producing NK cells have also been reported for a sepsis model of Streptococcus pyogenes. Although, in this case, the NK cell subpopulation and the signalling molecules were not studied in detail, this suggests that similar mechanisms are involved in sepsis by Gram-positive bacteria.

Together, the inventors have demonstrated that the excess of IFN-γ produced during infection by the Gram-positive intracellular bacteria L. monocytogenes is detrimental. It renders the host susceptible to sepsis. Therefore, use of blocking antibodies or small molecule inhibitors against IFN-γ or CD27/CD70 signalling is a possibility to interfere with sepsis caused by Gram-positive bacteria.

In contrast, different molecular mechanisms are responsible for sepsis elicited by Gram-negative bacterial infection. Thus, the establishment of a distinct treatment will be required. However, over-stimulation of NK cells via CD27/CD70 might be a way to interfere with acute or chronic viral diseases such as influenza or HIV and HCV, where aberrant numbers of CD27 positive cells, but elevated levels of CD70 were found.

Using monoclonal antibodies to specific stimulatory molecules the inventors activate a subset of NK cells, that are shown to be extremely important for the clearance from the influenza infection. The activation of this subset of cells will bring beneficial effects against influenza infections and will improve host survival.

The following figures, sequences, and examples merely serve to illustrate the invention and should not be construed to restrict the scope of the invention to the particular embodiments of the invention described in the examples. All references cited in the text are hereby incorporated in their entirety by reference.

### Figures

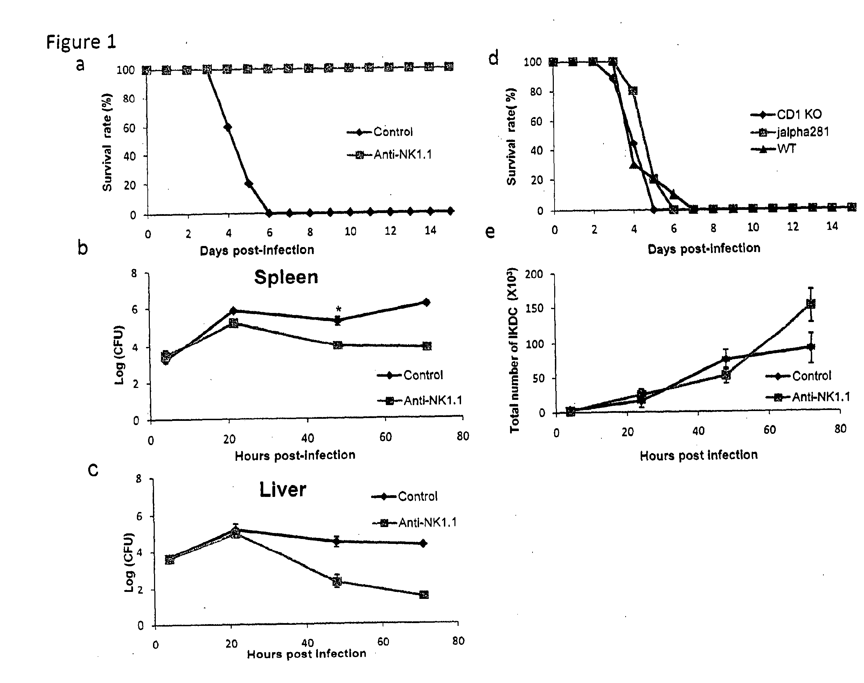

Figure 1 shows the depletion ofNK cells ameliorate murine listeriosis. (a) Survival curve of mice injected with anti-NK1.1 antibody (anti-NK1.1) or an irrelevant antibody (control) 24 hours before i.v. infection with 5 x 10⁴ L. monocytogenes. (b) Colony forming units (CFU) of L. monocytogenes in spleen and (c) liver of infected mice depleted with anti-NK1.1 or control mice at different time points post infection. The depletion of NK cells resulted in highly significant reduction of the bacterial burden in the spleen and on the liver of the depleted animals (two way ANOVA, with results F = 74.65, DFn = 3, DFd = 32, P < 0.0001, and F = 92.86, DFn = 3, DFd = 29; P < 0.0001). (d) Survival curve of mice infected with 5 x 10⁴ L. monocytogenes deficient for CD1 (CD1 ko) or Jα281 (jalpha281) compared to wild type mice (WT). (e) The influence of anti-NK1.1 injection on the population of supposedly splenic IKDC was determined by analyzing the percentage of B220⁺CD111c⁺DX5⁺GR1⁻ cells at different time points post infection. The antibody treatment had no significant effects on the IKDC population for at least 48 hrs. Statistical analysis was carried out using two way ANOVA (F = 2.40, DFn = 1, DFd = 24, P = 0.1346).

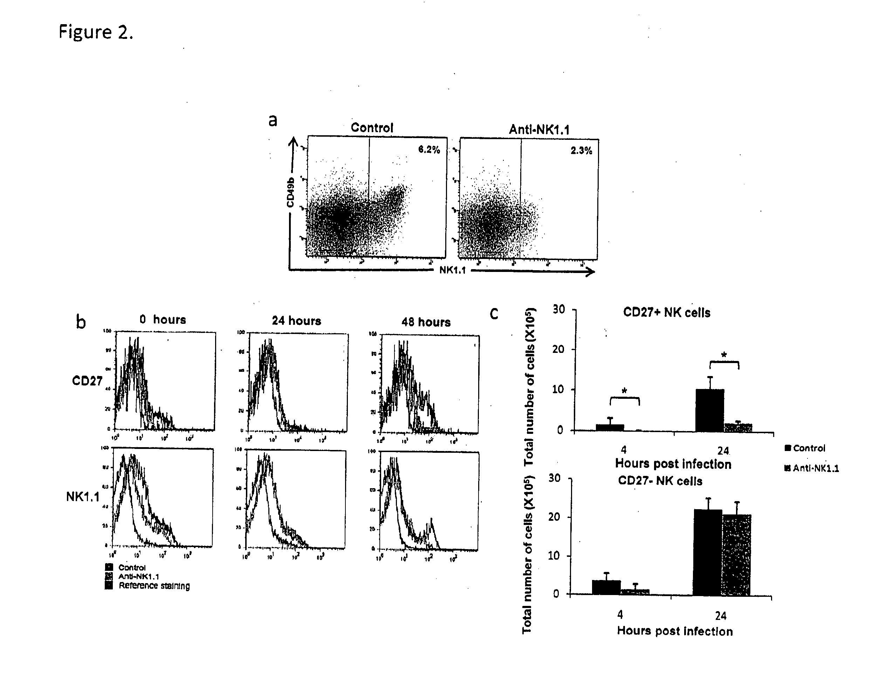

Figure 2 shows that CD27^{hi} NK cells are the major subset of NK cells depleted by anti-NK1.1 antibody. (a) Flow cytometry of CD3⁻ splenocytes from mice treated 24 hours before with anti-NK1.1 or control antibody. (b) Histograms from CD3-NK1.1⁺ splenocytes stained for the NK cell surface markers CD27 and NK1.1 at different time points PI. Autofluorescence is showed in blue. . (c) Total cell number of splenic CD27^{hi} and CD27^{lo} NK cell subpopulations after infection of depleted or control mice. * indicates significant differences between the CD3⁻NK1.1⁺CD27⁺ population in control and anti-NK1.1 treated mice (two tailed student test with P≤ 0.01). Negligible differences for CD3⁻NK1.1⁺CD27⁻ cells were found between the two groups of mice.

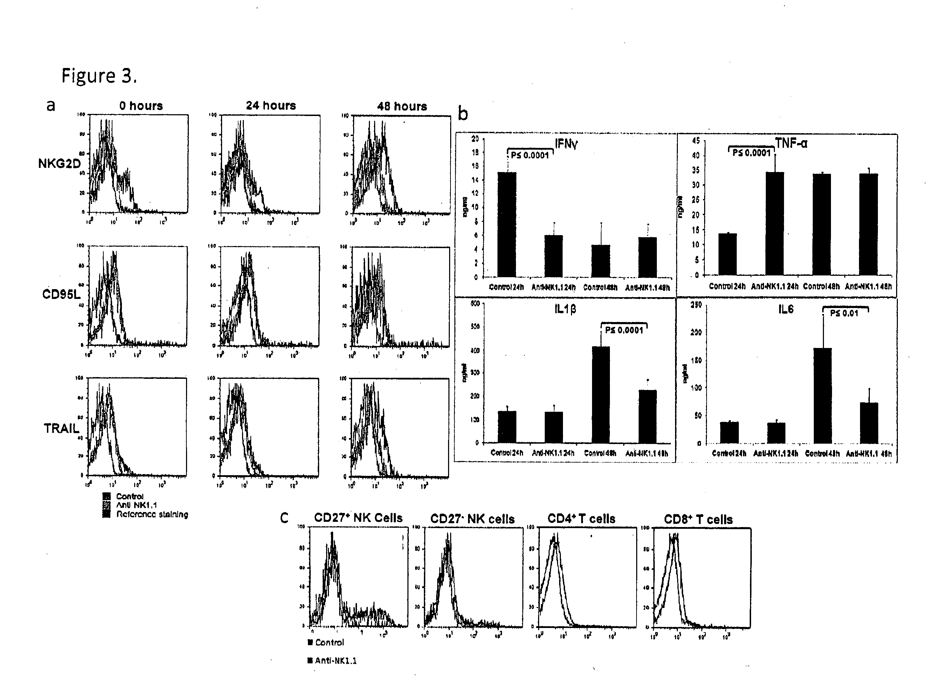

Figure 3 shows that CD27^{hi} NK cells produce large amounts from IFN-γ after stimulation. (a) NKG2D and the death ligands TRAIL and CD95L expression on CD3⁻NK1.1⁺ splenocytes. The expression of NKG2D was always significantly different along the measured time points post infection between the control and anti-NK1.1 depleted mice (P≤ 0.0002, P≤ 0.0006 and P≤ 0.0001 respectively for the 0, 24 and 48 hours post infection). The expression of TRAIL was only significantly different at the time point 0 and 48 hours post infection, with the respective P≤ 0.0001 and P≤ 0.0001. The autofluorescence is showed in blue. (b) The concentration of cytokines in the serum of infected mice was determined for IFN-γ, IL-6 and IL-1β by sandwich ELISA and TNF-α by an in vitro reporter assay. The P values are indicated in the diagrams. (c) Flow cytometry for intracellular IFN-y production by different populations of splenocytes. The amount of IFN-γ produced by CD27⁺ cells of control mice is highly superior compared to CD27⁺ cells from anti-NK1.1 treated mice (P≤ 0.0002) or CD27⁻ NK cells from control mice (P≤ 0.0001). All the statistical analyses were done using a non-parametric two-tailed student test.

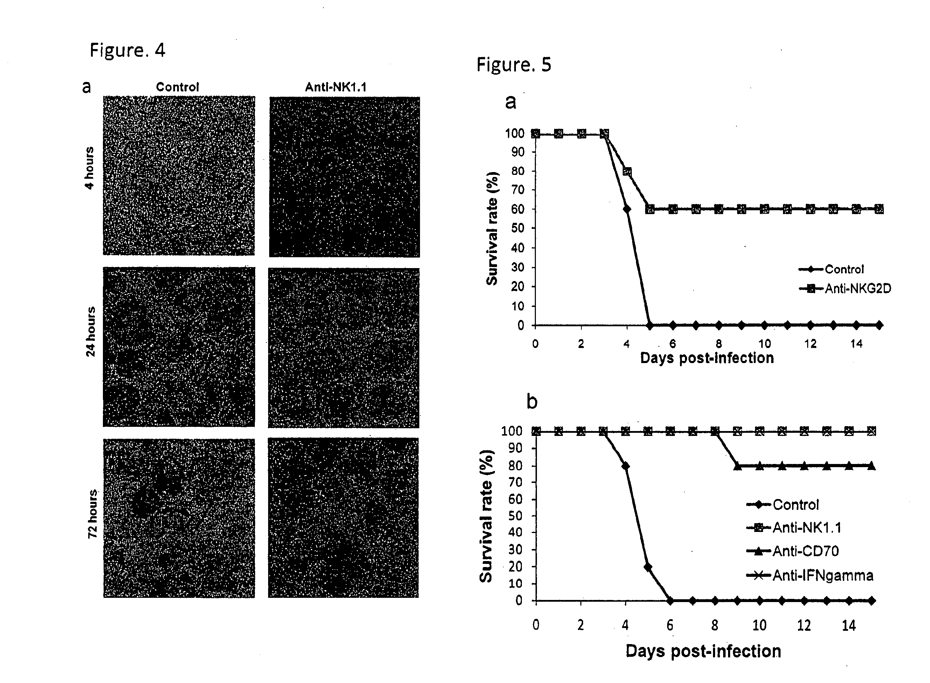

Figure 4. Parafine sections from the spleen of mice infected with L. Monocytogenes at different time points post-infection. On the control mice is clearly visible, at later time points, large areas of necrotic tissue. This areas are not present on the mice treated with anti-NK1.1.

Figure 5 shows the depletion of the IFN-γ or the blocking of the stimulating events leads to the effect that this cytokine production improves the host survival during sepsis. (a) Survival curves from mice infected with L. monocytogenes. (a) mice treated with control antibody and anti NKG2D (b) Mice were 24 hours previously treated with isotype antibody (control) and NK1.1 antibody (anti-NK1.1), at the moment of infection with blocking antibody against CD70 (anti-CD70), and 10 hours post infection with neutralizing antibody against IFN-γ (anti-IFN-γ).

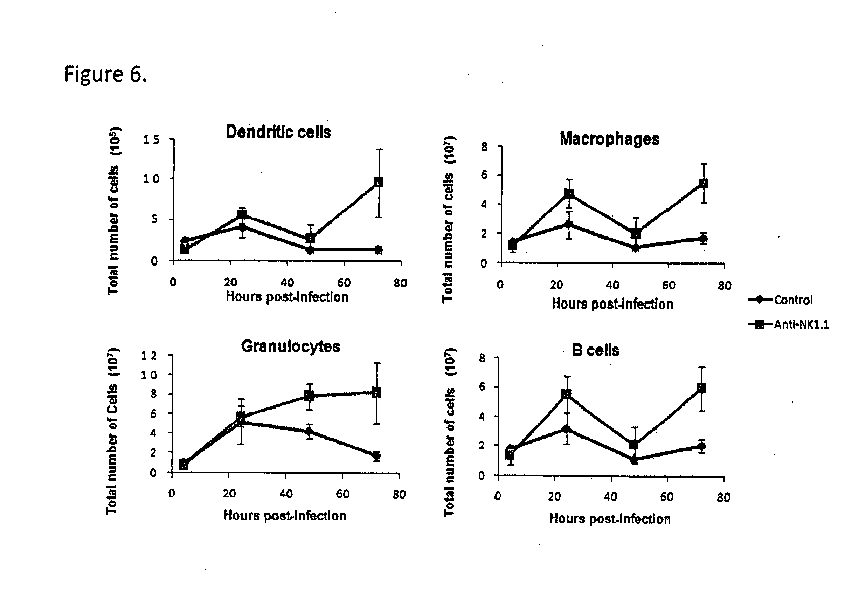

Figure 6 shows that mice with higher levels of IFNy on the serum have lower number of viable lymphocytes on the spleen but no increase on the apoptosis levels. Mice treated with control antibody or anti-NK1.1 antibody were studied for total number of viable splenic subpopulations (annexin V negative, PI negative) at different times post-infection. The treatment with anti-NK1.1 antibody has a extremely highly significant effect on the increase of B cells, macrophages and PMN on the spleen. A two way ANOVA was performed and p ≤ 0.0001, F = 10.20, DFn = 3, DFd = 31, and p ≤ 0.0002, F = 9.14, DFn = 1, DFd = 32, respectively for B cells and PMN.

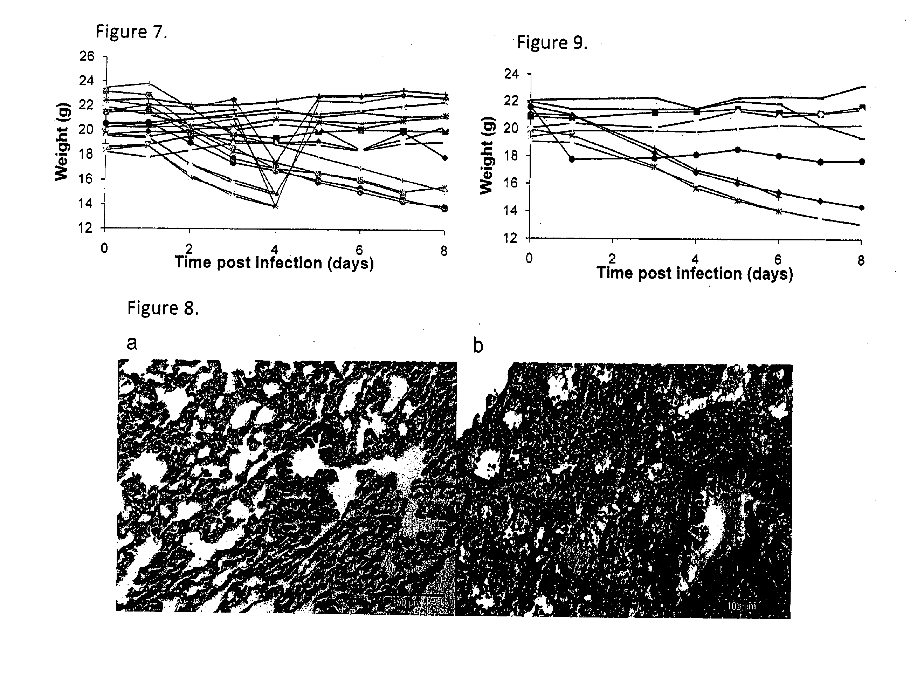

Figure 7 shows the weight loss of control mice (blue lines) or mice depleted for NK cells (red lines) after infection with influenza virus.

Figure 8 shows the haemotoxylin/eosin staining from paraffin sections of 8 weeks old female C57BL/6 mice infected with a sublethal dose from influenza after pre-treatment with (a) control isotype antibody (b) CD27highNK1.1 high depleting antibody.

Figure 9 shows the weight loss of control mice (red lines) or mice stimulated with anti-CD27 antibody (blue lines) after infection with influenza virus.

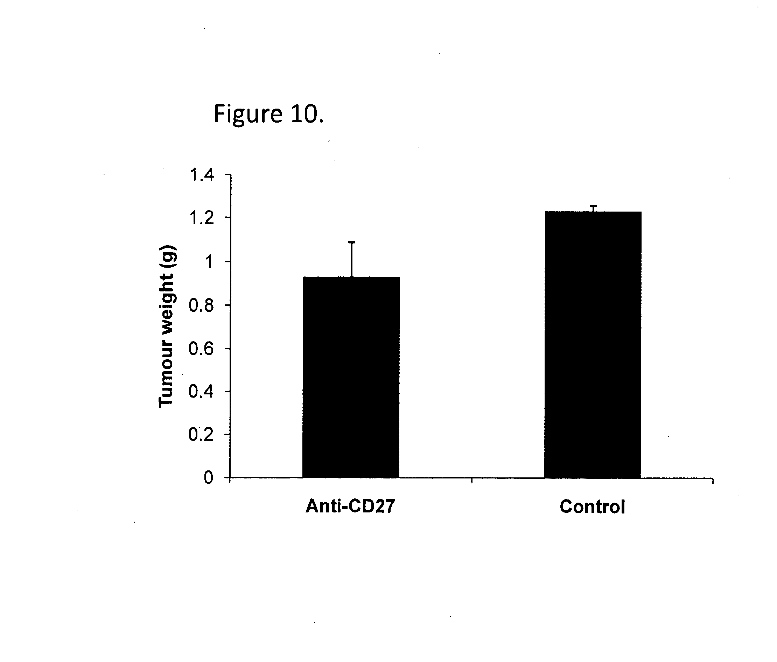

Figure 10 shows that a single I.V. injection of NK cell activating antibody anti-CD27 was sufficient to significantly reduce the tumour size of a B16 melanoma on W.T. mice. The statistical analyses was performed with a Students t-Test and p<0,03.

### Examples

### Methods

Bacteria: A dose of 2 x 10⁴ L. monocytogenes strain EGDe (serovar 1/2a) in PBS was injected i.v. into the mice.

Mice: C57B1/6 mice were obtained from Harlan (Borchem) and maintained in pathogen free conditions. CD1^{-/-} and Jα281^{-/-} were kindly provided by Ulrich Schaible, Max Plank Institute for Infection Biology, Berlin. All the mice used were females between 8 to 14 weeks of age.

In vivo antibody injection: All the antibodies where azid free and were injected i.v. into the target mice. The in vivo depletion of NK cells was achieved by injecting the mice 24 hours before the infection with 100µg of the anti-NK1.1 antibody (clone PK136, self-produced). Neutralization of IFN-γ was achieved by injecting 200µg of the antibody clone AN-18 (self produced) into mice 12 hours after infection. The blocking of CD70 was done by injecting 100µg of the antibody clone FR70 (eBiosciences) at time 0h of infection. The control group was injected with the isotype control rat IgG (Dianova) at the same time point and at the same concentration as the functional antibody.

Antibodies and Flow cytometry: The data acquisition was performed on an FACS Canto (BD) and the analyses on FACS Diva and FloJo software. Extracellular staining: Live splenocytes were gated as PI negative. The antibodies anti-TRAIL (N2B2), anti-B220 (RA3-6B2), CD11c (N418), CD49b (DX5), CD11b (M1/70), Grl (Ly.6G), CD27 (LG.7F9), NKG2D (CX5), CD3 (17.A2), CD95L (MFL3) from eBiosciences; NK1.1 (PK136) self made and CD19 (1D3) from BD were used for staining of splenocytes.

Apoptosis: Recent apoptotic splenocytes were quantified using the Annexin V assay kit from AbD Serotec according to the manufacturer instructions.

Intracellular staining: Splenocytes were collected at different time post-infection and incubated with brefeldin A (Fluka) for 4h at 37°C. No PMA/lonomycin was used since it could give false positive results. The intracellular staining was performed using the antibodies anti-granzyme B (eBiosicences) and anti-IFN-γ together with the kit cytofix/cytoperm from BD, according to the manufacturer instructions.

Histology: At several time points post-infection, the spleen was collected and fixed in formaldehyde for paraffin sections. Paraffin sections of 3µm were stained with Haematoxilin/Eosin and analyzed in an Olympus BX51.

### Results

The activation ofNK cells via CD27 leads to improved Influenza clearance

The inventors also found that the correct activation of NK cells via the membrane bound receptors NKG2D, NK1.1 and CD27 is fundamental for development of a correct immune response and survival of the host against the intracellular bacteria Listeria monocytogenes. This subset of CD27highNK1.1high NK cells was described by other group to be able to secrete large amounts of cytokines after stimulation, to be able to rapidly migrate in response to inflammatory cytokines and to be present on normal conditions in the lung (Hayakawa et al. 2006; Hayakawa and Smyth 2006).

To start studying the role of CD27highNK1.1high NK cells during influenza infections, the inventors decided to infect mice previously depleted for this NK cell subset and a control group of mice. The depletion was performed injecting depleting antibodies against the CD27highNK1.1high subset of NK cells 24h before the sublethal influenza infection. The depletion resulted on an approximately 80% reduction of the CD27highNK1.1high NK cell subset from the internal organs, for several days after antibody injection. The infection was performed intranasally, with a sublethal dose from influenza virus at day 0 of infection. The two groups of mice were controlled and weighted for a time period of 8 days post infection. After this period of time the mice were euthanized and the internal organs analysed by flow cytometry and histology. According to our results, mice depleted for the CD27highNK1.1high subset of immunoregulatory NK cells drastically lost weight along the post-infection time when compared with the control group of mice (Fig. 7).

At the moment of termination of the experiment, 40% of the NK cells depleted mice had succumbed to the influenza infection in comparison with only 10% from the control group (data not shown).

The analyses of the lungs by immunohistochemistry from the euthanized mice depleted for NK cells, revealed alveolar injury, usually manifested by the loss of alveolar epithelial cells, oedema, haemorrhage, hyaline membrane formation, and inflammation in alveolar septae and alveolar space (Fig 8b). The lungs from the group of control mice did not revealed any signs of infection or inflammation, revealing that by day 8 the virus had been clean by the immune system (Fig 8a). In this way the inventors can state that the population of CD27highNK1.1high NK cell subpopulation is essential for the priming of an adequate immune response against influenza infections.

After infection of mice with a lethal dose of Influenza, the activation of CD27highNK cells improves the resistance of these mice against the virus (Fig. 9). The majority of the control mice only treated with isotype antibody lost rapidly weight after influenza infection and died from it.

Using monoclonal antibodies we can stimulate only NK cells and induce their proliferation and IFN-gamma production. Proliferation and IFN-γ production can be enhanced by the simultaneous costimulation of CD27, NK1.1, NKG2D and NCR. The pleiotropic cytokine IFN-gamma promotes the resistance against viral infection through the activation of macrophages and dendritic cells.

The manipulation of NK cells via CD27/CD70 may also represent a means to interfere in a different way in ither acute or chronic viral infections such as HIV and HCV, were aberrant numbers of CD27 positive cells and elevated levels of CD70 are found (Wolthers et al, 1996). In fact data from Matter et al (Nolte et al 2006) suggests that CD27 signaling favors persistence of LCMV infection. The blocking of this signaling pathway resulted in the production of specific antibodies by B cells that facilitated the viral clearance.

Depletion of NK1.1⁺ cells ameliorates infection by Listeria monocytogenes Cells positive for the cell surface marker NK1.1 are detrimental to the outcome of infection by L. monocytogenes. However, NK1.1 is known to be expressed by NK and NKT cells as well as by the recently described interferon killer dendritic cells. In order to test, which population is responsible for the detrimental effect, the inventors depleted NK1.1 cells by injecting an anti-NK1.1 monoclonal antibody. After 24 hrs, the animals were challenged i.v. with a lethal dose of L. monocytogenes (2 x 10⁴). Using these conditions, the inventors could confirm that the presence of NK1.1⁺ cells was detrimental to the survival of mice. All mice injected with the control antibodies died within 6 days, whereas all mice that had received depleting anti-NK1.1 survived the infection (Fig. 1a). This was also reflected in the colony counts from spleen and liver of animals treated in the same way (Fig. 1 b, c). Interestingly, until 24 hrs no difference can be observed between the two groups, but already after 48 hrs, a 10-100-fold decrease of bacterial numbers is found in mice injected with the anti-NK1.1 antibody, compared to control animals (Fig. 1 b, c).

To unravel which of three different NK1.1⁺ cell populations is responsible for the detrimental effect during Listeria infection, the inventors first concentrated on NKT cells. NKT cells are T cells that co-express NK cell markers together with a semi invariant or invariant T cell receptor (TCR) that recognizes microbial or self glycolipids presented by the non-polymorphic CD1d molecules. NKT cells can be classified according to their TCR variance into Type I or Type II NKT cells (Kronenberg 2005).

By employing mice deficient for CD1d that would lack type I and II NKT cells or mice deficient for Jα281 lacking only Type I NKT cells we wanted to establish the relevance of such NKT cells for murine listeriosis. The mice were infected with a lethal dose of L. monocytogenes, and compared to wild type mice. Results displayed in Fig 1d demonstrate that recombinant mice do not profit from the absence of any type of NKT cells. Both types of mice succumb to the Listeria infection like the wild type. This suggests that under our conditions, NKT cells are not responsible for the detrimental effects exhibited by NK1.1⁺ cells, in agreement with data obtained by Tupin et al. before (Tupin, Kinjo, and Kronenberg 2007).

A second possibility was the involvement of IKDC. IKDC are a recently discovered type of DC that exhibit the cytotoxic characteristics of natural killer cells, like expression of CD49b, and are able to present antigen like dendritic cells (Chan et al. 2006;Taieb et al. 2006). These cells have recently been claimed to be activated NK cells. Nevertheless, the inventors studied the presence of cells with the described characteristics during Listeria infection in the spleen of control mice or mice treated with anti-NK1.1.

The level of NK1.1 on the surface of supposedly IKDC is comparatively low (Fig. 2a). Obviously, this expression level is not sufficient to render the IKDC sensitive to antibody treatment. Similar numbers were observed in the spleen of control and anti-NK1.1 treated mice during the early time points of infection (Fig. 1e). In addition, the numbers increased almost in parallel in both mice within the observation time. This strongly suggests that the supposedly IKDC are not responsible for the positive effect of depletion of NK1.1⁺ cells during Listeria infection.

Together, from these data it can be concluded that neither NKT cells nor IKDC are responsible for the detrimental role of NK1.1⁺ cells in murine listeriosis. Rather, sensu stricto natural killer cells play the negative role.

Anti NK1.1 antibodies deplete NK1.1^{hi} CD27^{hi} NK cells

Under the experimental conditions, 63% of NK1.1⁺ cells were depleted 24 hours after anti-NK1.1 antibody treatment (Fig. 2a). However, depletion was restricted to NK1.1⁺ cells. The NK1.1^{lo} and NK1.1^{int} populations remained almost unchanged by this treatment (Fig. 2b, lower panel).

Murine NK cells can be classified according to CD27 expression into two subsets with distinct effector and migratory capacity (Hayakawa et al. 2006). CD27 belongs to the tumor necrosis factor receptor (TNFR) family and functions as a co-stimulator on NK, T and B cells (Borst, Hendriks, and Xiao 2005). Murine CD27^{hi} NK cells are potent cytokine producers with high migratory capacity that also exhibit strong cytotoxicity (Hayakawa et al. 2006; Hayakawa and Smyth 2006).

In order to determine, how the two NK cell populations are affected by the antibody depletion, the inventors analyzed control and depleted mice for the presence of CD27^{hi} NK cells and their behavior during the early phase of Listeria infection. As can be seen in Fig. 2b, NK1.1⁺ cells strongly increased during Listeria infection in non-depleted mice while little change is observed in depleted mice. In parallel, the CD27^{hi} population increased during infection in control mice, a population that was absent in the antibody treated group.

This was corroborated by comparing the CD3⁻NK1.1⁺CD27⁺ and the CD3⁻NK1.1⁺CD27⁻ cell population during this period of infection in control and depleted mice. Both populations drastically increase during Listeria infection in normal mice (Fig 2c). Interestingly, only the CD27⁺ population is affected by depletion with NK1.1 antibodies while negligible changes became obvious for the CD27⁻ population (Fig 2c). This strongly suggests that the detrimental effect exerted by NK cells during murine listeriosis is most likely due to NK1.1^{hi} CD27⁺ cells.

CD27^{hi} NK cells produce high amounts of IFNγ after infection
One of the activating receptors of NK cells is NKG2D (CD314), a type II cell surface glycoprotein. It is stimulated by ligands that are up-regulated on stressed cells. When splenic NK cells of infected mice were tested for the expression of NKG2D, the inventors found a strong expansion of such cells in control mice while these cells were almost absent in mice depleted with NK1.1 antibodies (Fig. 3a).

Upon stimulation, NK cells are able to control infections by inducing apoptosis of infected cells. Several effector mechanisms could be employed. For instance, production of cytokines like IFN-γ and TNF-α, engagement of membrane-bound death ligands like CD95L/FasL and TRAIL or release of perforin and granzymes that activate the caspase pathway.

The membrane bound receptors are apparently not responsible for the negative effect of NK cells on the course of Listeria infection. The expression of CD95L/FasL and TRAIL on the surface of NK cells in control and NK1.1 depleted mice is similar (Fig. 3a). Thus, NK1.1^{hi} as well as NK1.1^{lo} cells should be able to induce apoptosis via such molecules. The same is true for granzyme B expression, which is unaltered by depletion i.e. is equally expressed by NK1.1^{hi} and NK1.1^{lo} cells (data not shown).

Interestingly, in control mice increase in NKG2D expressing NK cells was associated with an increase in IFN-γ production (Fig. 3b). Serum of such mice contained more IFN-γ than the serum of NK1.1 depleted animals. These higher levels of IFN-γ resulted in higher levels of IL-12 in serum of control mice. IL-12 is normally released by IFN-γ stimulated macrophages and dendritic cells. IL-12 then could feed back and stimulate NK cells to produce more IFN-γ.

Since the main difference between the control and the depleted mice is the CD27^{hi} NK cells population, the inventors investigated whether these cells could be the producers of IFN-γ during the early Listeria infection. Indeed, a high percentage of CD27^{hi} NK cells from control mice produced significant intracellular amounts of IFN-γ, compared to CD27^{lo} NK cells or CD4 and CD8 T cells (Fig. 3c).

Interestingly, serum concentration of the pro-inflammatory cytokine TNF-α was significantly increased by 24 hrs post infection in the serum of NK1.1 depleted mice (Fig. 3b). This cytokine is known to activate effector ells but also for its chemoattractive effect on neutrophils. The importance of TNF-α for survival of listeriosis is well established. Mice that lack this cytokine or its receptor rapidly succumb to infection by L. monocytogenes.

Based on these findings, the inventors postulate that an overstimulation of the subset of NK cells, possibly via the co-stimulation of several activating surface molecules like NK1.1, NKG2D or CD27 itself, results on an excessive production of IFN-γ and a delayed production of TNF-α that is detrimental for early listerial control.

Inhibition of IFN-γ or blockage CD27 ligand CD70 rescues mice infected with L. monocytogenes A prediction of this hypothesis would be that inhibiting IFNy should ameliorate infection by L. monocytogenes. This was indeed the case. Mice that were treated 10 hrs after infection with a neutralizing antibody against IFN-γ all survived a lethal infection by L. monocytogenes, like mice treated with anti NK1.1 or anti NKG2D whereas all control mice died (Fig.5a and b).

Since CD27 can act as costimulatory receptor, the inventors further wanted to see whether interaction of CD27 with its ligand CD70 is responsible for the increase in production of IFN-γ.

We, therefore blocked CD70 by injecting an anti-CD70 antibody at the time of infection. This resulted in the survival of almost all of the mice. Testing the serum of such mice revealed that as hypothesized blocking the CD27/CD70 interaction resulted in an abolishment of the high concentration of IFN-γ in infected mice.

Depletion of NK1.1hi cells increases numbers of splenic myeloid cells during listeriosis
IFN-γ is able to induce apoptosis of infected cells. On the other hand, it is also able to suppress random and directed migration of neutrophils. Neutrophils together with macrophages are the major innate effector cells that migrate into the spleen during listeriosis. The inventors therefore determined the kinetics of myeloid and lymphoid cells during listeriosis in the spleen of control mice and mice depleted with anti-NK1.1. As can be seen in Fig. 6, depletion of NK1.1^{hi} cells had a strong effect on the numbers of neutrophils, macrophage and B cells and to some extend also on dendritic cells. In particular the numbers of neutrophils declined drastically in control mice after 24hrs of infection, while in depleted mice the number stayed constant. These cells could control the early stage of infection.

The lower numbers of effector cells in the spleen of untreated infected mice could be due to induction of apoptosis by IFN-γ, as has been proposed. However, analysis of apoptotic cells in the spleen of both types of mice after Listeria by flow cytometry did not reveal any differences (data not shown). Thus, either the cells do not undergo apoptosis, or apoptotic bodies are removed very quickly. Alternatively, the cells analyzed are not able to migrate into the infected spleen. Such effect has been described as activity of IFN-γ.

Massive death of myeloid cells in spleen is inhibited by anti-NK1.1 treatment
The histological analysis revealed a massive cell death in the spleen of mice not depleted for NK1.1 (Fig. 4a). By 72 hrs post infection a massive destruction of the spleen with vast necrotic areas becomes obvious. In contrast, in infected mice that are depleted with anti-NK1.1 rearrangements of the follicles are obvious but the general splenic structure remains intact. As a consequence of accumulation of dead cells in the spleen the induction of anti-inflammatory cytokines like IL-6 (Fig. 3b) and IL-10 could take place that was found in high concentrations 48 and 72 hours post-infection in the serum of mice treated with control antibody.

Reference List
Arens, R. et al. Constitutive CD27/CD70 interaction induces expansion of effector-type T cells and results in IFNgamma-mediated B cell depletion. Immunity. 2001 Nov; 15(5): 801-12.

Borst, J., J. Hendriks and Y. Xiao CD27 and CD70 in T cell and B cell activation. Curr Opin Immunol. 2005 Jun; 17(3): 275-81.

Buras, J. A., B. Holzmann and M. Sitkovsky Animal models of sepsis: setting the stage. Nat Rev Drug Discov. 2005 Oct; 4(10): 854-65.

Carrero, J. A., B. Calderon and E. R. Unanue Lymphocytes are detrimental during the early innate immune response against Listeria monocytogenes. J Exp Med. 2006 Apr 17; 203(4): 933-40.

Chan, CW et al. Interferon-producing killer dendritic cells provide a link between innate and adaptive immunity Nat Med. 2006 Feb; 12(2): 207-13.

Delano, M. J. et al. MyD88-dependent expansion of an immature GR-1(+)CD11b(+) population induces T cell suppression and Th2 polarization in sepsis. J Exp Med. 2007 Jun 11; 204(6): 1463-74.

Geerdes, HF et al. Septicemia in 980 patients at a university hospital in Berlin: prospective studies during 4 selected years between 1979 and 1989. Clin Infect Dis. 1992 Dec; 15(6): 991-1002.

Hayakawa, Y. et al. Functional subsets of mouse natural killer cells. Immunol. Rev. 214 (2006): 47-55.

Hayakawa, Y. and M. J. Smyth. CD27 dissects mature NK cells into two subsets with distinct responsiveness and migratory capacity. J. Immunol. (2006) 176(3): 1517-24.

Kronenberg, M. Toward an understanding of NKT cell biology: progress and paradoxes. Annu. Rev. Immunol. 23 (2005): 877-900.

Kuranaga, N. et al. A defective Th1 response of the spleen in the initial phase may explain why splenectomy helps prevent a Listeria infection. Clin Exp Immunol. 2005 Apr; 140(1): 11-21.

Linde-Zwirble, WT and DC Angus. Severe sepsis epidemiology: sampling, selection, and society. Crit Care. 2004 Aug;8(4):222-6.

Lorente, JA and JC Marshall. Neutralization of tumor necrosis factor in preclinical models of sepsis. Shock. 2005 Dec; 24 Suppl 1: 107-19.

Minino, AM, MP Heron and BL Smith. Deaths: preliminary data for 2004. (2006) Natl. Vital Stat. Rep. 54(19): 1-49.

Nolte, M.A. and R.A. van Lier. 2006. The price of the CD27-CD70 costimulatory axis: you can't have it all. J.Exp.Med. 203:2405-2408.

Pamer, EG Immune responses to Listeria monocytogenes. Nat. Rev. Immunol. (2004) 4(10): 812-23.

Riedemann, NC, RF Guo and PA Ward. Novel strategies for the treatment of sepsis." Nat. Med. 9(5) (2003): 517-24.

Taieb, J. et al. A novel dendritic cell subset involved in tumor immunosurveillance. Nat. Med. 12(2) (2006): 214-19

Teixeira, HC and SH Kaufmann. Role of NK1.1+ cells in experimental listeriosis. NK1+ cells are early IFN-gamma producers but impair resistance to Listeria monocytogenes infection. J Immunol. 1994 Feb 15; 152(4): 1873-82

Tupin, E., Y. Kinjo and M. Kronenberg. The unique role of natural killer T cells in the response to microorganisms. Nat. Rev. Microbiol. 5(6) (2007): 405-17.

Van Amersfoort, ES, TJ Van Berkel and J. Kuiper. Receptors, mediators, and mechanisms involved in bacterial sepsis and septic shock. Clin. Microbiol. Rev. 16(3) (2003): 379-414.

van der Poll T. Immunotherapy of sepsis. Lancet Infect. Dis. 1(3) (2001): 165-74.

Vazquez-Boland, JA et al. Listeria pathogenesis and molecular virulence determinants. Clin. Microbiol. Rev. 14(3) (2001): 584-640.

Vincent, JL, Q. Sun and MJ Dubois Clinical trials of immunomodulatory therapies in severe sepsis and septic shock." Clin. Infect. Dis. 34(8) (2002).

Wolthers, K.C., S.A. Otto, S.M. Lens, D.N. Kolbach, R.A. van Lier, F. Miedema, and L. Meyaard. 1996. Increased expression of CD80, CD86 and CD70 on T cells from HIV-infected individuals upon activation in vitro: regulation by CD4+ T cells. Eur.J.Immunol. 26:1700-1706.

## Claims

1. Use of a ligand of CD27 for the preparation of a medicament for the prevention or treatment of cancer, viral and bacterial infections as well as immune disorders through the activation, depletion or inhibition ofNK cells.

2. Use according to claim 1, wherein said ligand is activating NK cells, and is for the prevention or treatment of viral infections, for example influenza virus.

3. Use according to claim 2, wherein said activation of said NK cells furthermore promotes the efficient clearance of the virus from the host system.

4. Use according to claim 1, wherein said ligand is depleting or inhibiting NK cells, and is for the prevention or treatment of diseases selected from the group of an infection caused by Gram-positive bacteria, such as Listeria monocytogenes, the treatment or onset of diabetes, graft versus host disease, immune disorders, sepsis, and viral infections, for example LCMV.

5. Use according to any of claims 1 to 4, wherein said ligand is selected from a CD27-specific antibody, an CD27-binding fragment thereof, a CD27-binding peptide, and a CD27-interacting substance.

6. Use according to claim 5, wherein said antibody is a human, humanized, mouse or chimeric antibody.

7. Use according to any of claims 1 to 6, wherein said ligand is administered systemically and/or administered locally.

8. Use according to any of claims 1 to 7, wherein the ligand is for administration in combination with other chemotherapeutically active substances, such as antibiotics or anti-cancer chemotherapeutics.

9. A method for screening for CD27 ligands comprising the steps of:
a) incubating a cell expressing CD27 with a putative ligand,
b) measuring, if a binding between CD27 and said putative ligand occurs,
c) in the case of a binding of said ligand to CD27 is measured, measuring, if said binding between CD27 and said identified ligand also leads to a CD27-mediated activation, depletion or inhibition ofNK cells.

10. The method according to claim 9, wherein said screening takes place in vitro or in vivo.

11. The method according to claim 9 or 10, wherein said ligand is selected from a peptide library, a combinatory library, a cell extract, in particular a plant cell extract, a "small molecular drug", a bacterial metabolite, a phage display, an antibody or fragment thereof, a protein and/or a protein fragment.

12. Method for the production of a pharmaceutical formulation, comprising the steps of:
a) performing a method according to any of claims 9 to 11, and
b) formulating the identified ligand for CD27 with a pharmaceutically acceptable carrier and/or excipient.

13. A screening tool for a ligand for CD27 for treating or preventing cancer, viral and bacterial infections as well as immune disorders through the activation, depletion or inhibition ofNK cells, wherein said tool is an NK cell which is recombinantly expressing CD27.

14. A screening tool for a ligand for CD27 for treating or preventing cancer, viral and bacterial infections as well as immune disorders through the activation, depletion or inhibition ofNK cells, wherein said tool is a non-human transgenic mammal whose NK cells express CD27.

15. A pharmaceutical composition for treating or preventing cancer, viral and bacterial infections as well as immune disorders through the activation, depletion or inhibition of NK cells, obtainable by a method according to claim 12.

16. A method for treating or preventing cancer, viral and bacterial infections as well as immune disorders through the activation, depletion or inhibition of NK cells, comprising administering to a subject in need thereof an effective amount of a pharmaceutical composition according to claim 15.
